(19) **Europäisches Patentamt / European Patent Office / Office européen des brevets**

(11) **EP 4 544 287 B1**

(12) **FASCICULE DE BREVET EUROPEEN**

(45) Date de publication et mention de la délivrance du brevet:
**11.03.2026  Bulletin 2026/11**

(21) Numéro de dépôt: **23734301.7**

(22) Date de dépôt: **26.06.2023**

(51) Classification Internationale des Brevets (IPC):
**G01N 15/0205** *(2024.01)*    **G01N 15/06** *(2024.01)*
**G01N 21/53** *(2006.01)*      **G01N 33/18** *(2006.01)*
**G01N 21/47** *(2006.01)*      **G01N 15/00** *(2024.01)*
**G01N 15/02** *(2024.01)*

(52) Classification Coopérative des Brevets (CPC):
**G01N 21/538; G01N 15/0211; G01N 15/06; G01N 33/1873;** G01N 15/075; G01N 2015/0046; G01N 2015/0294; G01N 2021/4711

(86) Numéro de dépôt international:
**PCT/EP2023/067308**

(87) Numéro de publication internationale:
**WO 2024/002965 (04.01.2024 Gazette 2024/01)**

(54) **SYSTÈME DE DÉTERMINATION DE L'ATTÉNUATION D'UNE ONDE LUMINEUSE TRAVERSANT UN VOLUME D'ÉCHANTILLONNAGE**

SYSTEM ZUR BESTIMMUNG DER DÄMPFUNG EINER LICHTWELLE, DIE DURCH EIN ABTASTVOLUMEN FLIESST

SYSTEM FOR DETERMINING THE ATTENUATION OF A LIGHT WAVE PASSING THROUGH A SAMPLING VOLUME

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorité:  **27.06.2022  FR 2206413**

(43) Date de publication de la demande:
**30.04.2025  Bulletin 2025/18**

(73) Titulaires:
• **Centre National de la Recherche Scientifique**
  **75016 Paris (FR)**
• **Université Clermont Auvergne**
  **63000 Clermont-Ferrand (FR)**

(72) Inventeurs:
• **COUTRIS, Pierre**
  **63530 Volvic (FR)**
• **FEBVRE, Guy**
  **15000 Aurillac (FR)**
• **FOURNOL, Jean-François**
  **63830 Durtol (FR)**
• **BOUCHER, William**
  **63122 Ceyrat (FR)**

(74) Mandataire: **IPAZ**
  **Bâtiment Platon**
  **Parc Les Algorithmes**
  **91190 Saint-Aubin (FR)**

(56) Documents cités:
**US-A1- 2009 103 085**

• **AUSTIN R W AND PETZOLD T J: "An Instrument For The Measurement Of Spectral Attenuation Coefficient And Narrow Angle Volume Scattering Function Of Ocean Waters", PROC. SPIE 0064, OCEAN OPTICS IV, vol. 0064, 1975, US, pages 50 - 61, XP093023095, ISSN: 0277-786X, ISBN: 978-1-5106-4548-6, DOI: 10.1117/12.954493**

## Description

## Domaine technique

L'invention se rapporte à un système de détermination de l'atténuation d'une onde lumineuse traversant un volume d'échantillonnage, ainsi qu'à un procédé de détermination de l'atténuation d'une onde lumineuse traversant un volume d'échantillonnage.

Les nuages ont un rôle prépondérant dans le bilan radiatif terrestre du fait de la forte interaction entre les hydrométéores (gouttelettes d'eau et/ou cristaux de glace) qui les constituent avec le rayonnement solaire et tellurique.

L'amélioration de la connaissance des interactions nuage-rayonnement et la compréhension du rôle des différents nuages dans le système climatique nécessite de bien connaitre leurs propriétés optiques et microphysiques afin d'implémenter dans les modèles atmosphériques des propriétés qui soient représentatives des nuages.

L'extinction (ou l'épaisseur optique qui en découle) est une propriété optique fondamentale. Elle caractérise l'atténuation d'une onde lumineuse traversant un milieu du fait des processus d'absorption et de diffusion. Elle est souvent décrite à l'aide du coefficient d'extinction (volumique), noté $\beta$ et définit comme suit :

$$\beta(\lambda) = c_{abs}(\lambda) + c_{sca}(\lambda)$$

Où $\lambda$ est la longueur d'onde de l'onde incidente, $c_{abs}$ est le coefficient d'absorption (en $m^{-1}$), et $c_{sca}$ est le coefficient de diffusion (en $m^{-1}$).

Dans le cadre de l'invention, il est considéré, pour simplifier les équations, que $c_{abs} = 0$ et donc que le coefficient d'extinction $\beta(\lambda) = c_{sca}(\lambda))$ et la dépendance à la longueur d'onde sera omise ( $\beta(\lambda) = \beta$) en considérant le système comme étant monochromatique.

Le coefficient de diffusion est défini à partir de la section efficace volumique $\mu(\theta, \varphi)$ de diffusion par l'équation suivante :

$$c_{sca} = \int_{4\pi} \mu(\theta, \varphi) \sin(\theta)\, d\theta d\varphi$$

Dans cette équation, $\theta \in [0 - \pi]$ et $\varphi \in [0 - 2\pi]$ sont respectivement l'angle polaire et l'angle azimutal.

Lorsque le volume diffusant contient une population de particules de tailles $D \in [D_{min} - D_{max}]$ caractérisée par une distribution dimensionnelle N(D), la section efficace volumique de diffusion est alors donnée par :

$$\mu(\theta, \varphi) = \int_{D_{min}}^{D_{max}} \left(\sigma_{sca}(\theta, \varphi, D)\right) * N(D) dD$$

Où N(D) dD est la concentration en particules de tailles comprises entre *D* et *D* + dD (en $m^{-3}$), $\sigma_{sca}(D)$ est la section efficace de diffusion moyenne des particules de tailles comprises entre *D* et *D* + dD (en $m^2$).

La caractérisation des propriétés optiques des nuages de glace à la microstructure complexe, du fait de la très grande variabilité horizontale et verticale des propriétés physiques (taille, forme, masse, présence d'inclusions, rugosité de surface, section efficace de diffusion) des hydrométéores qui les constituent, compte parmi les défis scientifiques actuels et sur ce point, la mesure in situ, c'est à dire réalisée dans les nuages même, présente un enjeu majeur dans ce domaine.

Dans l'atmosphère, l'extinction optique peut-être estimée de différentes manières. Par exemple, à l'aide de moyens de télédétection active, comme le lidar CALIOP embarqué sur le satellite CALIPSO, l'extinction peut être déduite de la mesure de puissance rétrodiffusée moyennant des hypothèses fortes sur le coefficient de diffusion multiple et/ou le rapport lidar qui dépendent du type de cible (aérosol, gouttelette, cristaux). Par ailleurs, cette technique ne fonctionne qu'au travers de milieux optiquement fins dans lesquels l'atténuation du faisceau laser n'est pas trop élevée.

D'autres instruments, pouvant être embarqués sur des plateformes de mesures aéroportées, tels que des spectromètres optiques ou encore des néphélomètres polaires, peuvent être utilisés. Toutefois, ces instruments ne permettent de réaliser une mesure in situ directe. L'extinction est déduite des mesures de granulométrie ou d'indicatrices de diffusion, moyennant des hypothèses fortes, ce qui induit une forte incertitude.

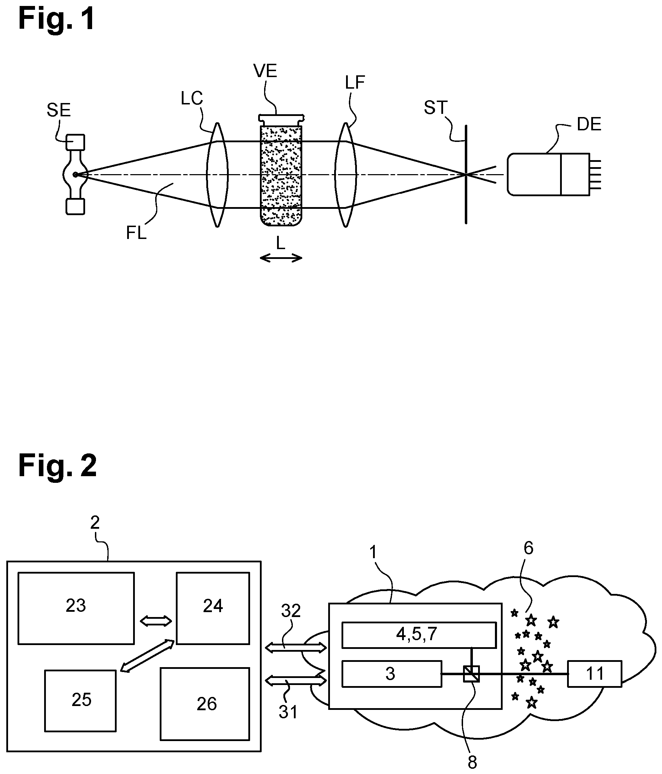

Pour mesurer l'extinction dans un volume de façon directe, on utilise généralement un transmissiomètre, dispositif radiométrique grâce auquel l'atténuation d'un faisceau lumineux traversant un volume de nuage est mesurée. Le principe est illustré par la figure 1. Un faisceau lumineux FL d'intensité incidente $I_0$ est émis par une source d'émission SE, et collimaté par une lentille de collimation LC. Le faisceau traverse un volume d'échantillonnage VE caractérisé par une extinction volumique $\beta$ et une épaisseur L. L'intensité transmise I est collectée à l'aide d'une lentille de focalisation LF puis mesurée à l'aide d'un détecteur DE après un passage par un sténopé ST.

L'atténuation du faisceau est décrite par la loi de Beer-Bouguer-Lambert :

$$I/I_0 = \exp(-\beta L)$$

Où I et $I_0$ correspondent respectivement à l'intensité incidente et à l'intensité transmise (en $W.m^{-2}$), $\beta$ correspond au coefficient d'extinction volumique (en $m^{-1}$), et L correspond à la longueur géométrique au travers du milieu échantillonné (en $m$ ).

La détermination du coefficient d'extinction par application de la loi de Beer-Bouguer-Lambert suppose donc de pouvoir mesurer l'intensité transmise, c'est à dire les photons qui n'ont pas interagi par diffusion ou absorption avec les hydrométéores. Il est donc nécessaire de pourvoir différencier la lumière réellement transmise sans interaction avec les particules de celle qui a été diffusée vers l'avant, ce qui est très difficile à réaliser en pratique.

Plusieurs types d'arrangements optiques ont été proposés pour réaliser cette mesure de transmission, mais tous ont un champ de vision non nul, caractérisé par le rapport entre le diamètre du sténopé ST (non nul) et la distance focale de la lentille LF (non infiniment grande). Le détecteur de ce genre de dispositif intercepte donc une fraction du rayonnement diffusé par les particules dans un secteur angulaire proche avant, c'est-à-dire très proche de la direction de propagation de l'onde incidente.

Puisque le détecteur intercepte une fraction de l'énergie diffusée par les particules, celle-ci s'additionne à l'énergie transmise, et fausse la mesure de transmission. Le coefficient d'extinction déduit à partir de la mesure de transmission non corrigée est donc systématiquement sous-estimé. L'erreur commise, intrinsèque au principe de mesure des transmissiomètres, dépend des caractéristiques optiques du dispositif de mesure de transmission (son champ de vision), et des propriétés optiques de la population de particules observées (section efficace volumique ou coefficient de diffusion et fonction de phase). Elle est particulièrement importante dans le cas d'objet diffusant très fortement la lumière vers l'avant, tel que les cristaux constituant les nuages de glace.

La demande de brevet US 2009/103085 A1 concerne un nouveau type de spectrophotomètres destinés à caractériser des matériaux turbides en déterminant leurs paramètres optiques, à savoir le coefficient d'absorption, le coefficient de diffusion, le facteur d'anisotropie et l'indice de réfraction réel, en fonction de la longueur d'onde dans le spectre d'intérêt.

Le document « An Instrument For The Measurement Of Spectral Attenuation Coefficient And Narrow Angle Volume Scattering Function Of Ocean Waters » (Austin, R. W. et T. J. Petzold, Proc. SPIE 0064, Ocean Optics IV, 10 novembre 1975) présente un instrument qui peut être immergé, afin d'étudier l'atténuation optique dans l'océan. L'instrument effectue une mesure de transmission dans le volume d'échantillonnage, puis trois mesures de la section efficace volumique selon trois angles polaires proches de l'axe optique (4, 8 et 16 mrad, 0 mrad indiquant la direction de propagation de la lumière)

L'article de Austin et Petzold décrit le dispositif permettant de réaliser successivement ces quatre mesures grâce à un mécanisme de roue comprenant une ouverture circulaire (sténopé de la mesure de transmission) et trois ouvertures annulaires de tailles différentes définissant les champs de vision des trois mesures de diffusion.

Cependant, l'instrument présenté dans cet article présente l'inconvénient d'effectuer les mesures de transmission et de diffusion de façon séquentielle. En appliquant cet instrument à de la caractérisation des hydrométéores présents dans un nuage en mouvement, le fait de procéder à des mesures séquentielles fausserait la mesure. En effet, le volume d'échantillonnage ne serait pas identique d'un instant de mesure à l'autre.

Un autre inconvénient du prototype présenté dans l'article est que les angles auxquels sont mesurés la diffusion sont limités en nombre (trois dans l'article) et fixes car déterminés mécaniquement par la taille des ouvertures annulaires. Ils ne peuvent donc pas être modifiés par l'utilisateur au cours de la mesure et/ou en post-traitement.

De plus, dans l'article de Austin et Petzold, la section efficace volumique n'est pas résolue selon l'angle azimutal $\varphi$ : l'utilisation d'un masque annulaire et d'un détecteur monoélément produit une mesure de l'intensité diffusée intégrée entre 0 et $2\pi$.

Par ailleurs, l'instrument est particulièrement adapté au milieu aqueux ; l'atténuation y est beaucoup plus élevée que dans l'atmosphère, donc les mesures peuvent être probantes même avec une longueur du volume d'échantillonnage de l'ordre de un ou deux mètres entre la source d'émission et les voies de réception. Ainsi, dans l'article un entretoise est utilisé pour maintenir l'alignement entre la partie émission et la partie réception.

Dans le cas de milieux moins atténuant, dans la plupart des nuages de l'atmosphère terrestre par exemple, il serait nécessaire de fortement augmenter la longueur entre la source d'émission et les voies de réception. Une entretoise de plusieurs mètres, suffisamment rigide pour maintenir l'alignement optique sur une telle longueur, étant difficilement envisageable, il serait alors nécessaire de prévoir des mécanismes d'alignement précis, augmentant ainsi la complexité et le coût du système.

Pour des mesures d'extinction optique dans l'atmosphère, il serait nécessaire de fortement augmenter la longueur entre la source d'émission et les voies de réception. Une entretoise de plusieurs mètres étant difficilement envisageable, il serait alors nécessaire de prévoir des mécanismes d'alignement précis, augmentant ainsi la complexité et le coût du système.

Il existe ainsi un besoin pour des systèmes et des procédés de détermination de l'atténuation d'une onde lumineuse traversant un volume d'échantillonnage, qui permettent d'effectuer les mesures transmission et de diffusion proche de l'axe optique pour tous types de milieu, et de façon coïncidente et colocalisée., et de cartographier la section efficace volumique sur un large intervalle d'angles polaires et azimutaux avec une résolution angulaire suffisante dans les deux dimensions (polaire et azimutale).

**Résumé de l'invention**

Un objet de l'invention est donc un système de détermination de l'atténuation d'une onde lumineuse traversant un volume d'échantillonnage, comprenant :

- une partie mesure comprenant :

   -- une source d'émission d'un faisceau optique collimaté ;

   -- une voie de mesure de référence, configurée pour mesurer une intensité optique de référence émise par la source d'émission ;

   -- une voie de mesure de transmission, configurée pour mesurer une intensité optique transmise dans le volume d'échantillonnage et diffusée dans le volume d'échantillonnage dans un intervalle d'angles polaires compris entre 0° et un angle de collection $\theta_{coll} > 0$, l'angle polaire étant défini rapport à l'axe optique du faisceau optique collimaté, où 0° indique la direction de propagation ;

   -- une voie de mesure de diffusion, configurée pour mesurer une intensité optique diffusée dans le volume d'échantillonnage, à une pluralité d'angles polaires par rapport à l'axe optique du faisceau optique collimaté compris entre $\theta_{coll}$ et un angle de détection maximal $\theta_{max} > \theta_{coll}$, afin de caractériser les propriétés du volume d'échantillonnage dans un secteur angulaire proche avant ;

   -- un ensemble de guidage, configuré pour guider le faisceau optique collimaté de la source d'émission vers les voies de mesure au travers du volume d'échantillonnage ;

- une partie pilotage, configurée pour contrôler les voies de mesure pour effectuer les mesures de transmission et de diffusion de façon concomitante, et pour déterminer l'atténuation du faisceau optique collimaté dans le volume d'échantillonnage en fonction de l'intensité optique de référence, de l'intensité optique transmise et diffusée, et de l'intensité optique diffusée.

Avantageusement, l'ensemble de guidage comprend une pluralité de séparateurs de faisceaux pour transmettre le faisceau optique collimaté vers la voie de mesure de référence, vers la voie de mesure de transmission et vers la voie de mesure de diffusion, et au moins un rétroréflecteur optique, disposé le long de l'axe optique de la source d'émission, et configuré pour replier sur lui-même le faisceau optique ayant traversé le volume d'échantillonnage en direction de la source d'émission.

Avantageusement la voie de mesure de diffusion comprend une lentille objectif de diffusion et un capteur optoélectronique multiélément configuré pour imager le plan de Fourier de la lentille objectif de diffusion.

Avantageusement, un élément absorbant, configuré pour absorber le faisceau optique transmis et diffusé entre 0° et $\theta_{coll}$, recouvre les pixels de la partie centrale du capteur optoélectronique multiélément.

Avantageusement, l'élément absorbant a un taux d'absorption qui décroit depuis la partie centrale du capteur optoélectronique multiélément vers les extrémités du capteur optoélectronique multiélément.

Avantageusement, le capteur optoélectronique multiélément est dépourvu d'éléments d'acquisition optique dans sa partie centrale.

Avantageusement, le capteur optoélectronique multiélément a une dynamique suffisamment élevée pour mesurer l'intensité transmise et pour mesurer l'intensité diffusée.

Avantageusement, le capteur optoélectronique multiélément est un capteur CMOS.

Avantageusement, la voie de mesure de diffusion comprend un occulteur disposé dans le plan focal de la lentille objectif de diffusion et un montage composé d'au moins deux lentilles, configuré pour imager le plan de Fourier de la lentille objectif de diffusion sur le capteur optoélectronique multiélément au travers d'un diaphragme de Lyot.

Avantageusement, la voie de mesure de transmission comprend une lentille objectif de transmission, et un sténopé de rayon $r_t$ placé à la distance focale $f_t$ de la lentille objectif de transmission, où $\tan(\theta_{coll}) = r_t/f_t$.

Avantageusement, la partie pilotage est configurée pour :

- déterminer les valeurs de sections efficaces volumiques de diffusion du volume d'échantillonnage à partir de l'intensité optique diffusée dans le volume d'échantillonnage pour différents angles par rapport à l'axe optique du faisceau optique collimaté ;

- appliquer un ajustement de courbe aux valeurs de sections efficaces volumiques de diffusion, et extrapoler la courbe obtenue aux valeurs de sections efficaces volumiques de diffusion entre 0° et $\theta_{coll}$ ;

- déterminer le coefficient d'extinction déduit de la mesure de transmission contaminée par la diffusion avant $\beta^*$ avec la relation suivante :

$$\beta^* = \beta - \int_0^{2\pi} \int_0^{\theta_{coll}} \mu(\theta, \varphi)\sin(\theta)\,d\theta\,d\varphi$$

Où $\beta$ correspond au coefficient d'extinction réelle calculée par la loi de Beer-Bouguer-Lambert, $\mu(\theta, \varphi)$ correspond à la valeur de sections efficaces volumiques qui varie en fonction de l'angle polaire $\theta$ et de l'angle azimutal de rotation autour de l'axe l'optique $\varphi$

Avantageusement, la source d'émission, la voie de mesure de référence, la voie de mesure de transmission et la voie de mesure de diffusion sont intégrées dans un même boitier.

L'invention se rapporte aussi à un procédé de détermination de l'atténuation d'une onde lumineuse traversant un volume d'échantillonnage, comprenant au moins une itération consistant à effectuer :

- une émission d'un faisceau optique collimaté par une source d'émission ;

- une mesure d'une intensité optique de référence émise par la source d'émission ;

- une mesure d'une intensité optique transmise dans le volume d'échantillonnage et diffusée dans le volume d'échantillonnage dans un intervalle d'angles polaires compris entre 0° et un angle de collection $\theta_{coll} > 0$, l'angle polaire étant défini rapport à l'axe optique du faisceau optique collimaté, où 0° indique la direction de propagation ;

- une mesure d'une intensité optique diffusée dans le volume d'échantillonnage, à une pluralité d'angles polaires par rapport à l'axe optique du faisceau optique collimaté compris entre $\theta_{coll}$ et un angle de détection maximal $\theta_{max} > \theta_{coll}$, afin de caractériser les propriétés du volume d'échantillonnage dans un secteur angulaire proche avant ;

- un contrôle des mesures réalisées de façon concomitante, et une détermination de l'atténuation en fonction de l'intensité optique de référence, de l'intensité optique transmise, et de l'intensité optique diffusée.

Avantageusement, le procédé comprend une pluralité d'itérations.

Avantageusement le volume d'échantillonnage est un environnement nuageux, en particulier un nuage en phase de glace.

## Description des figures

D'autres caractéristiques, détails et avantages de l'invention ressortiront à la lecture de la description faite en référence aux dessins annexés donnés à titre d'exemple.

La figure 1, déjà décrite, illustre le principe de la mesure de transmission dont on déduit ensuite l'extinction par application de la loi de Beer-Bouguer-Lambert..

La figure 2 illustre l'architecture du système selon l'invention.

La figure 3 est un schéma de principe de la partie mesure du système.

La figure 4 est une illustration de la voie de mesure de transmission.

La figure 5 est une illustration d'un mode de réalisation de la voie de mesure de diffusion.

La figure 6 illustre une vue de face d'un élément absorbant selon l'invention.

La figure 7 est une illustration d'un mode de réalisation de la voie de mesure de diffusion selon un mode de réalisation de l'invention.

L'invention est décrite en référence à la figure 2, qui illustre le système de façon très schématique.

Le système comprend une partie mesure 1 comprenant les modules d'émission et de détection nécessaires à la mesure, et d'une partie pilotage 2 via laquelle un opérateur contrôle l'instrument et l'acquisition des données.

La partie pilotage 2 comprend notamment une sous-partie d'alimentation électrique 26 pour alimenter la partie mesure 1 et la partie pilotage 2 en énergie électrique. Une liaison électrique 31 relie la sous-partie d'alimentation électrique 26 à la partie mesure 1.

La partie pilotage 2 comprend également une sous-partie de contrôle commande et d'acquisition 24 pour rassembler l'ensemble des paramètres intervenants dans la mesure et pour les fournir à la partie mesure 1 via une liaison informatique externe 32. La sous-partie de contrôle commande et d'acquisition 24 a aussi pour rôle le contrôle des éléments de la partie mesure 1, le recueil de leurs statuts et la gestion des modes opératoires du système. Elle peut aussi acquérir des informations de servitudes et de surveillance (par exemple les températures de fonctionnement ou les tensions d'alimentation), et gérer des alarmes avec réaction immédiate et/ou informatives.

Les paramètres de mesure et les paramètres d'environnement sont sauvegardés dans une sous-partie de stockage 25, reliée informatiquement à la sous-partie de contrôle commande et d'acquisition 24. Les paramètres sont enregistrés dans un format exportable et exploitable à l'aide de matériel et logiciels informatiques.

Une sous-partie d'interface homme-machine 23, reliée informatiquement à la sous-partie de contrôle commande et d'acquisition 24, permet à un utilisateur d'interagir avec les différents éléments de mesure, et de visualiser ou prévisualiser les résultats de mesure, en vue leur exploitation. Les données affichées peuvent être par exemple des graphiques représentant les séries temporelles des puissances émises et reçues ou encore la mesure d'atténuation. La sous-partie d'interface homme-machine 23 peut aussi permettre à l'utilisateur de démarrer et arrêter la mesure, de contrôler la modification des paramètres de mesures, ou encore d'afficher les statuts des modules du système, les paramètres de mesure et d'environnement, et les alarmes et différentes informations de sécurité.

La partie mesure 1 comprend une source d'émission 3 d'un faisceau optique collimaté, une voie de mesure de référence 4, une voie de mesure de transmission 5, une voie de mesure de diffusion 7, et ensemble de guidage pour guider le faisceau optique collimaté de la source d'émission 3 vers les voies de mesure (4, 5, 7) au travers du volume d'échantillonnage 6. Le volume d'échantillonnage 6 peut être, par exemple, un volume d'échantillonnage atmosphérique, ou un volume d'échantillonnage aquatique, ou tout autre milieu dans lequel une mesure d'extinction optique doit être effectuée.

La partie mesure 1 est exposée au volume d'échantillonnage 6, et la partie pilotage 2 peut être soit exposée, en partie ou en totalité, au volume d'échantillonnage 6, ou être située en dehors du volume d'échantillonnage 6 pour un pilotage à distance.

Dans un mode de réalisation de l'invention, la partie mesure 1 peut se trouver à l'extérieur d'un bâtiment, par exemple sur le toit du bâtiment, et la partie pilotage 2 à l'intérieur du bâtiment. Dans un autre mode de réalisation, la partie mesure 1 peut se trouver en partie à l'extérieur d'un avion (sur le fuselage et/ou sous une aile), et la partie pilotage 2 peut être abritée dans la carlingue de l'avion.

La partie pilotage 2 contrôle la voie de mesure de référence 4, la voie de mesure de transmission 5 et la voie de mesure de diffusion 7 pour effectuer les mesures de transmission et de diffusion de façon concomitante, et pour déterminer l'atténuation du faisceau optique collimaté dans le volume d'échantillonnage 6 en fonction de l'intensité optique de référence $I_0$ mesurée par la voie de mesure de référence 4, de l'intensité optique transmise et diffusée $I_T$ mesurée par la voie de mesure de transmission 5, et de l'intensité optique diffusée $I_D$ mesurée par la voie de mesure de diffusion 7. Etant donné que toutes les mesures de diffusion et de transmission sont faites de façon concomitante et colocalisée, il est possible d'utiliser les valeurs de section efficace volumique mesurées par la voie de diffusion pour corriger la valeur de l'intensité optique transmise et diffusée $I_T$ mesurée par la voie de mesure de transmission 5 avant d'appliquer la formule de Beer-Bouguer-Lambert pour calculer l'extinction.

En effet, la concomitance des mesures de transmission et de diffusion ainsi que la colocalisation de leurs volumes échantillonnés permet ensuite de d'estimer par calcul la part de l'énergie diffusée dans ce qui est mesurée par la voie de mesure de transmission 5, ce qui améliore la précision de la détermination du coefficient d'extinction par rapport aux solutions de l'état de la technique.

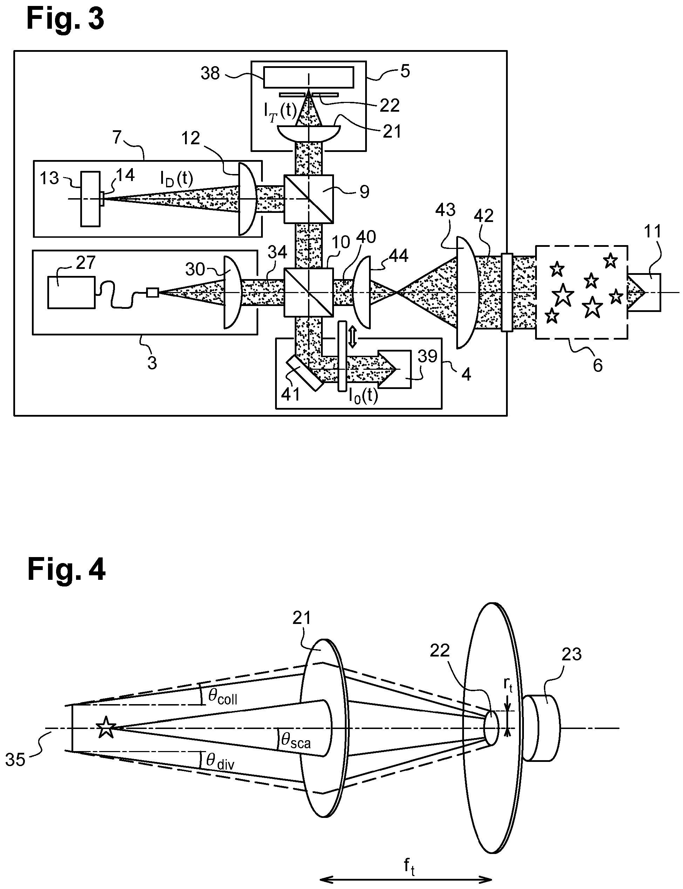

La figure 3 illustre la partie mesure 1 de façon plus détaillée. La source d'émission 3 comprend un composant opto-électronique 27 capable d'émettre de la lumière monochromatique, caractérisé par une longueur d'onde et une puissance optique. Par exemple, le composant opto-électronique 27 peut être une diode laser ayant une longueur d'onde $\lambda$ =808 nm et une puissance P$\geq$1 W. Le faisceau optique, après expansion, peut avoir un diamètre d' environ 45 mm, le diamètre devant être compatible avec les diamètres des éléments optiques de l'ensemble de guidage.

Les caractéristiques de la source d'émission 3 (longueur d'onde et puissance optique) peuvent être adaptées aux propriétés des particules diffusantes à caractériser (taille et concentration par exemple), de même que l'utilisation d'une source polychromatique, de filtres passe-bande et/ou de capteurs polychromatiques rendent possible la mesure de l'extinction à différentes longueurs d'onde dans des milieux où cela est pertinent.

Dans le cas où un laser est utilisé en tant que source d'émission, un isolateur peut être placé pour éviter une instabilité de la source provoquée par le retour de la lumière vers la source.

La source d'émission 3 comprend également une lentille de collimation 33, un iris pour limiter la largeur du faisceau, et éventuellement un système de bafflage interne.

Un premier séparateur de faisceaux 10 sépare le faisceau émis par la source d'émission 3 en un faisceau dirigé vers la voie de mesure de référence 4 et un faisceau dirigé vers le volume d'échantillonnage 6. La voie de mesure de référence 4 mesure la quantité d'énergie $I_0$ émise par la source d'émission éclairant le volume d'échantillonnage 6. La mesure de la quantité d'énergie $I_0$ peut être effectuée par un composant capable de capter un rayonnement du domaine optique et de le transformer en signal électrique, par exemple une photodiode. En variante, illustrée par la figure 3, la mesure de la quantité d'énergie $I_0$ peut être effectuée par le détecteur de la voie de transmission, par exemple une photodiode 38. Dans ce cas, la voie de mesure de référence 4 comprend un réflecteur 39 qui renvoie le faisceau optique vers la voie de mesure de transmission 5, ainsi qu'un obturateur 40 qui permet de sélectionner le flux envoyé vers la photodiode 38, ce flux étant soit celui de référence soit celui ayant interagi avec le volume d'échantillonnage. La sélection peut être opérée par translation de l'obturateur 40, ou par tout autre moyen permettant d'effectuer une telle sélection. Par commodité, le faisceau de la voie de mesure de référence 4 peut être replié à l'aide d'un miroir plan 41.

Le rapport du séparateur de faisceaux 10 peut être par exemple de 50/50. Cette valeur du rapport réflexion/transmission optimise l'énergie diffusée arrivant au détecteur dans un montage où le faisceau est replié à l'aide du coin de cube.

La largeur du faisceau optique collimaté 34 issu de la source est élargie par un doublet de lentilles 43, 44, puis traverse le volume d'échantillonnage 6, représenté sur la figure 3 par un environnement nuageux composé d'hydrométéores quelconques (ex. : gouttelettes d'eau liquide, cristaux de glace), représentés sous la forme d'étoiles. Le faisceau optique interagit avec les cristaux de glace, ce qui crée un phénomène de diffusion de la lumière vers l'avant, c'est-à-dire dans la direction de transmission du flux optique. Un hublot 42 permet d'effectuer l'interface entre l'intérieur du boitier et l'environnement extérieur.

Le flux optique est réfléchi par le séparateur de faisceaux 10, en direction d'un deuxième séparateur de faisceau 9, qui sépare le faisceau en deux parties. Le rapport du deuxième séparateur de faisceaux 10 peut être de 90/10 (90 % de la puissance du faisceau vers la voie de diffusion et seulement 10 % vers la voie de transmission). En effet, dans le cas où l'extinction est comprise entre 0,1 et 100 km$^{-1}$, la puissance transmise est bien plus grande que la puissance diffusée (plusieurs ordres de grandeur). Vu la puissance de la source, il y a plus d'énergie que nécessaire pour réaliser la mesure de transmission, donc le rapport du deuxième séparateur de faisceaux 10 permet d'envoyer le plus possible de puissance vers la voie de diffusion.

Un relais d'imagerie (non représenté sur les figures) peut être inséré entre les deux séparateurs de faisceau, afin d'imager le rétroréflecteur 11 sur les plans de détection (transmission et diffusion).

Une partie est transmise sur la voie de mesure de transmission 5. Dans cette voie, la quantité d'énergie transmise $I_T$ au travers du volume d'échantillonnage 6 d'épaisseur L et parvenant à un détecteur est comparée à l'énergie incidente $I_0$. Le détecteur 38 est composant capable de capter un rayonnement du domaine optique et de le transformer en signal électrique, par exemple une photodiode. Dans la pratique, on pourra utiliser la même photodiode pour mesurer $I_0$ et $I_T$.

L'énergie transmise $I_T$ est mesurée à l'aide d'un dispositif optique dont le principe est illustré par la figure 4. Le faisceau incident collimaté (avec une faible divergence, caractérisée par le demi-angle $\theta_{div}$) traverse le volume d'échantillonnage puis est collecté par une lentille objectif de transmission 21 après avoir traversé le volume d'échantillonnage et focalisé vers un détecteur, qui peut être une photodiode, au travers d'un dispositif de type sténopé 22 (ou trou d'aiguille) placé dans le plan focal de la lentille objectif de transmission 21.

Dans ce cas, le champ de vision du détecteur de transmission est un cône de demi-angle au sommet noté $\theta_{coll}$ (en général légèrement supérieur à $\theta_{div}$) dont la valeur est déterminée par le rayon du sténopé 22 (petite ouverture circulaire, « pinhole » en anglais), noté r, et par la focale de la lentille objectif de transmission 21, notée $f_t$, par la relation tan

$$\theta_{coll} = \frac{r_t}{f_t}$$

Ainsi, le détecteur 23 est capable de capter avec les objets diffusant du milieu échantillonné ainsi que les rayons diffusés dans un intervalle d'angles polaires compris entre 0° et $\theta_{coll}$, l'angle polaire étant défini rapport à l'axe optique 35 du faisceau optique collimaté 34, où 0° indique la direction de propagation, et $\theta_{coll}$ correspond à l'angle de collection de la mesure de transmission (en général de l'ordre du milliradian).

Comme cela est visible sur les figures 3 et 4, une partie de la lumière diffusée vers l'avant par les particules constituant le volume d'échantillonnage 6 est collectée par le détecteur 23 de la voie de mesure de transmission 5, et vient directement polluer la mesure de transmission, avec un angle de diffusion $\theta_{sca}$ compris entre 0° et $\theta_{coll}$ par rapport à l'axe optique du faisceau optique.

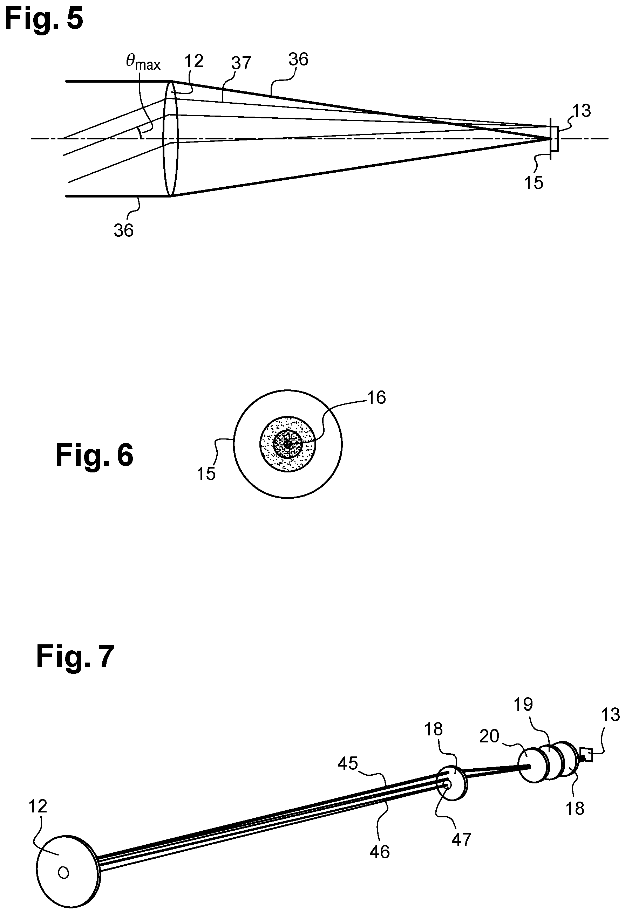

Afin de surmonter le problème de pollution de la mesure de transmission qui collecte une partie de l'intensité diffusée par les particules constituant le volume d'échantillonnage 6 en plus de l'intensité transmise, le système selon l'invention comprend une voie de mesure de diffusion 7, configurée pour mesurer une cartographie de la section efficace volumique de diffusion sur une plage d'angle azimutaux compris entre 0 et $2\pi$, et d'angles polaires par rapport à l'axe optique du faisceau optique collimaté 34 compris entre $\theta_{coll}$ et $\theta_{max}$, où $\theta_{max}$ correspond à un angle de détection maximal permettant de caractériser les propriétés du volume d'échantillonnage 6 dans un secteur angulaire proche avant (ou HFOV pour « Half Field Of View » ou demi-champ de vue du système de détection). Ainsi, $\theta_{max}$ est défini par la taille du capteur optoélectronique multiélément 13 et par la focale de la lentille objectif de diffusion 12 (cf. figure 5).

Cette cartographie, obtenue avec une résolution angulaire très fine, permet de définir une fonction de correction de la mesure de transmission à partir des quantités d'énergie diffusée mesurées en différents secteurs angulaires et de caractériser bon nombre de propriétés des hydrométéores échantillonnés (état thermodynamique, orientations privilégiée, taille et concentration).

Adaptées aux propriétés optiques des hydrométéores, et facilement adaptable à tous les écoulements diphasiques dans lesquels la diffusion du faisceau lumineux par les particules en suspension n'est pas négligeable, cette solution permet de réduire l'incertitude sur la détermination du coefficient d'extinction obtenu de la mesure de transmission.

Selon un mode de réalisation avantageux illustré par les figures 3 et 4, un réflecteur optique 11 est disposé le long de l'axe optique de la source d'émission 3. Il replie sur lui-même le faisceau optique 34 traversant le volume d'échantillonnage 6 en direction de la source d'émission 3.

L'utilisation d'un rétroréflecteur optique (réflecteur de type « coin de cube »), permet tout d'abord de replier le faisceau optique collimaté, réduisant ainsi l'encombrement pour une longueur optique donnée. Il est entendu que la loi de Beer-Bouguer-Lambert tient compte de la longueur totale du trajet optique dans le volume d'échantillonnage 6, dans les deux sens de transmission du faisceau.

Ceci permet également d'intégrer la source d'émission 3, la voie de mesure de référence 4, et les voies de mesure de transmission 5 et de diffusion 7 dans un seul et même boitier, ce qui facilite le maintien de l'alignement optique des différents modules de mesure avec la source durant les mesures, ainsi que l'installation et la maintenance du système.

Par ailleurs, l'utilisation d'un rétroréflecteur optique permet de préserver l'alignement optique entre la source d'émission 3 et les voies de mesure de transmission 5 et de diffusion 7, malgré la distance qui les sépare et les mouvements relatifs qui peuvent exister entre le boitier et le réflecteur. Dans le cadre de mesures d'extinction dans un milieu atmosphérique, une distance de plusieurs mètres (entre cinq et dix mètres) est généralement requise afin d'obtenir des mesures probantes. Le repliement du faisceau à l'aide d'un retroréflecteur permet de créer cette distance.

Dans les solutions de l'état de l'art, il est nécessaire de prévoir une structure mécanique suffisamment rigide sur une longueur de plusieurs mètres ou un mécanisme d'asservissement afin de maintenir un alignement parfait entre la source d'émission et les voies de mesure. Cela est d'autant plus vrai pour les applications aéroportées, compte tenu des vibrations qui peuvent se produire au niveau du fuselage. Les rayons étant par construction optique toujours réfléchis vers leur source (en raison des trois plans réfléchissants orthogonaux qui constituent le retroréflecteur), l'usage d'un rétroréflecteur simplifie l'installation et rend le dispositif tolérant à un désalignement du rétroreflecteur tout en permettant donc de se passer d'un mécanisme d'asservissement.

Toutefois, la présence d'un rétroréflecteur n'est pas essentielle à la mise en œuvre du système selon l'invention. Par exemple, pour des mesures de l'extinction dans des milieux pour lesquels la mesure peut être effectuée avec une longueur du volume d'échantillonnage de quelques dizaines de centimètres, ou pour des mesures effectuées en laboratoire, dans un environnement très stable, il peut être envisagé que la source d'émission et les voies de mesure soient positionnées en face l'une des autres. Elles seraient dans ce cas séparées par le volume d'échantillonnage.

Selon un mode de réalisation, la voie de mesure de diffusion 7 comprend une lentille objectif de diffusion 12 et un capteur optoélectronique multiélément 13 configuré pour imager le plan de Fourier de la lentille objectif de diffusion 12. Par ailleurs, la voie de mesure de diffusion doit permettre de cartographier la section efficace volumique à partir de l'intensité la lumière diffusée dans le volume d'échantillonnage aux angles polaires compris entre $\theta_{coll}$ et $\theta_{max}$ en tenant compte du fait que la puissance transmise et diffusée entre 0° et $\theta_{coll}$ arrive également sur cette voie de mesure et peut être très largement supérieure aux valeurs de puissance diffusée à mesurer.

Comme cela est visible sur les figures 3 et 5, la partie 36 du faisceau optique qui a été transmise au détecteur 23 de la voie de mesure de transmission 5 n'est pas captée par le capteur optoélectronique multiélément 13.

Le fait de placer le capteur optoélectronique multiélément 13 à la distance focale $f_d$ de la lentille objectif de diffusion 12 et de maintenir cette dernière parfaitement alignée avec le faisceau transmis permet d'obtenir une correspondance entre la direction dans laquelle la lumière est diffusée dans le volume d'échantillonnage, caractérisée par les angles $\theta$ et $\varphi$, et la position radiale du pixel du capteur optoélectronique multiélément 13 qui mesure cette lumière diffusée (distance $r_d$ par rapport à l'endroit du capteur optoélectronique multiélément 13 qui coïncide avec l'axe optique), avec la relation : $r_d = f_d \cdot \tan(\theta)$.

L'utilisation d'un capteur multiélément permet de réaliser une mesure résolue angulairement selon $\varphi$, contrairement au système à anneaux d'Austin et Petzold 1975 qui réalise une mesure intégrée sur $[0 - 2\pi]$. Il s'agit d'un avantage de plus de l'utilisation d'un capteur multiélément. Les mesures indiquent dans quels milieux cette propriété est satisfaite et permettent dans le cas contraire de déduire des informations sur l'orientation et la forme des cristaux.

Ainsi, l'invention permet d'obtenir, en une prise de vue, une cartographie à fine résolution angulaire de la puissance lumineuse diffusée sur une plage d'angles polaires d'intérêts compris entre $\theta_{coll}$ et $\theta_{max}$ et d'angles azimutaux compris entre 0 et $2\pi$, par les particules qui atténuent le faisceau, en même temps que la mesure de transmission. L'invention permet de surmonter le problème de variabilité intra- et inter-nuage des paramètres constitutifs des hydrométéores (taille, forme, rugosité de surface, etc.) qui rend inutile toute tentative de corriger la mesure de transmission à l'aide de fonctions de correction théoriques et/ou déterminées empiriquement à partir de mesures réalisées en laboratoire sur des échantillons artificiels.

Le capteur optoélectronique multiélément 13 peut être un capteur CMOS (pour « Complementary Metal-Oxide-Semiconductor »). Les capteurs CMOS ont une vitesse de fonctionnement (cadence image) plus élevée que les autres capteurs optoélectroniques multiéléments, ce qui est avantageux pour les applications de mesure scientifique. Cependant, d'autres capteurs optoélectroniques, tels que les capteurs CCD (pour « Charge Coupled Device »), pourraient être utilisés pour mettre en œuvre l'invention. La résolution angulaire de la voie de mesure de diffusion 7 dépend de la résolution du capteur optoélectronique multiélément 13. Pour un capteur CMOS, une résolution angulaire nettement inférieure au milliradian peut être envisagée.

Sauf à avoir un capteur optoélectronique multiélément 13 ayant une dynamique suffisamment élevée pour mesurer l'intensité transmise et pour mesurer l'intensité diffusée à certains angles polaires par rapport à l'axe optique du faisceau optique collimaté 34 compris entre $\theta_{coll}$ et $\theta_{max}$, ou d'être dépourvu de pixels dans la partie centrale correspondant à des angles polaires par rapport à l'axe optique du faisceau optique collimaté 34 compris entre 0° et $\theta_{coll}$, il est nécessaire d'adjoindre un dispositif anti-éblouissement 14 au capteur optoélectronique multiélément 13.

Un dispositif anti-éblouissement 14 évite au capteur optoélectronique multiélément 13 d'être ébloui par faisceau optique qui est focalisé en son centre tout en permettant de mesurer l'intensité optique diffusée dans le volume d'échantillonnage. En effet, le rapport de puissance entre le signal de diffusion et le signal de transmission peut être supérieur à 1 pour $10^{-6}$. De préférence, le dispositif anti-éblouissement 14 a les mêmes dimensions que le sténopé 22, dans le plan perpendiculaire à l'axe optique 35.

Selon un premier mode de réalisation, illustré par la figure 6, le dispositif anti-éblouissement 14 comprend un élément absorbant 15 qui est collé sur la vitre de protection du capteur optoélectronique multiélément 13. L'élément absorbant 15, qui peut se présenter sous la forme d'un film, occulte les pixels de la partie centrale 16 du capteur optoélectronique multiélément, afin d'absorber le faisceau optique transmis et diffusé entre 0° et $\theta_{coll}$. Un film absorbant « AcktarBlack » (marque déposée), de la société « Acktar » (marque déposée), a une réflectance très faible, et peut donc convenir pour l'application envisagée.

L'élément absorbant 15 peut avoir un taux d'absorption qui décroît depuis la partie centrale 16 du capteur optoélectronique multiélément 13 vers les extrémités du capteur, selon un gradient prédéfini, comme l'illustre la figure 6. Cela permet de lisser l'intensité à mesurer sur la surface du capteur.

Selon une variante illustrée par la figure 7, la voie de mesure de diffusion 7 comprend un occulteur 47, placé juste devant la lentille 18 disposé dans le plan focal (plan de Fourier) de la lentille objectif de diffusion 12 et un système optique composé d'au moins deux lentilles (18, 19), configuré pour imager ce plan sur le capteur optoélectronique multiélément 13 au travers éventuellement d'un diaphragme de Lyot 20. Le relais d'imagerie réalisé par les lentilles 18 et 19 permet de placer le dispositif occultant 17 dans le plan de Fourier et d'imager ce plan à l'aide du capteur optoélectronique multiélément 13 positionné physiquement hors du plan de Fourier, et donc à distance du disque occultant. Cela apporte un degré de liberté par rapport au positionnement du capteur optoélectronique multiélément 13. Le faisceau incident 45 arrive sur la lentille 18 avec un angle inférieur à $\theta_{max}$, et se propage au travers du dispositif jusqu'au capteur optoélectronique multiélément 13. Le faisceau incident 46, qui correspond au faisceau transmis et diffusé à des angles inférieurs à $\theta_{coll}$, est bloqué par l'occulteur 47.

Un filtre passe-bande peut être utilisé dans une solution monochromatique pour garantir que seule la lumière issue de la source arrive au détecteur (pour filtrer la lumière du soleil dans une mesure in situ par exemple).

Cette variante permet d'éviter que le phénomène de diffraction sur les bords de l'élément occultant 17 et de la lentille objectif 12 ne vienne contaminer la mesure de l'intensité diffusée par le capteur optoélectronique multiélément 13.

La mesure de puissance diffusée sur une pluralité d'angles polaires par rapport à l'axe optique du faisceau optique collimaté permet de caractériser les sections efficaces volumiques de diffusion du milieu sur ces mêmes angles.

A partir de l'intensité optique diffusée $I_D$ dans le volume d'échantillonnage pour différents angles par rapport à l'axe optique du faisceau optique collimaté 34, et de l'intensité optique de référence $I_0$, les valeurs de sections efficaces volumiques de diffusion du volume d'échantillonnage 6 sont déterminées par une loi linéaire connue de l'homme du métier. Un ajustement de courbe correspondant aux valeurs de sections efficaces volumiques de diffusion (par exemple une interpolation des valeurs), puis l'extrapolation de la courbe obtenue par ajustement vers l'angle de collection de transmission, permet d'estimer la section efficace volumique de diffusion entre 0° et l'angle $\theta_{coll}$.

Il est ensuite possible de déterminer le coefficient d'extinction déduit de la mesure de transmission contaminée par la diffusion avant $\beta^*$ avec la relation suivante :

$$\beta^* = \beta - \int_0^{2\pi} \int_0^{\theta_{coll}} \mu(\theta, \varphi) sin(\theta) d\theta \, d\varphi$$

Où $\beta$ correspond au coefficient d'extinction réelle calculée par la loi de Beer-Bouguer-Lambert, $\mu(\theta, \varphi)$ correspond à la valeur de sections efficaces volumiques qui varie en fonction de l'angle polaire $\theta$ et de l'angle azimutal de rotation autour de l'axe l'optique $\varphi$

L'invention se rapporte également au procédé mis en œuvre par le système précité. Le procédé peut être exécuté de façon ponctuelle, ou comprendre une pluralité d'itérations dans le temps, de façon à suivre une évolution temporelle des grandeurs mesurées.

Le procédé est particulièrement adapté pour caractériser un environnement nuageux, en particulier un nuage en phase de glace. En effet, les nuages en phase de glace sont un exemple typique de milieu diphasique constitué de particules fortement diffusantes et de tailles bien supérieures aux longueurs d'onde du visible, pour lesquels la diffusion de la lumière, en particulier vers l'avant, ne peut être négligée.

Le procédé peut également trouver des applications industrielles, par exemple pour de la caractérisation d'écoulement diphasique, ou pour des analyses granulométriques, et pour la réalisation de mesures de visibilité en aéronautique.

## Revendications

1. Système de détermination de l'atténuation d'une onde lumineuse traversant un volume d'échantillonnage, comprenant :

   - une partie mesure (1) comprenant :

      -- une source d'émission (3) d'un faisceau optique collimaté (34) ;
      -- une voie de mesure de référence (4), configurée pour mesurer une intensité optique de référence émise par la source d'émission (3) ($I_0$) ;
      -- une voie de mesure de transmission (5), configurée pour mesurer une intensité optique transmise ($I_T$) dans le volume d'échantillonnage (6) et diffusée dans le volume d'échantillonnage (6) dans un intervalle d'angles polaires compris entre 0° et un angle de collection $\theta_{coll} > 0$, l'angle polaire étant défini rapport à l'axe optique du faisceau optique collimaté (34), où 0° indique la direction de propagation ;
      -- une voie de mesure de diffusion (7), configurée pour mesurer une intensité optique diffusée dans le volume d'échantillonnage ($I_D$), à une pluralité d'angles polaires par rapport à l'axe optique du faisceau optique collimaté (34) compris entre $\theta_{coll}$ et un angle de détection maximal $\theta_{max} > \theta_{coll}$, afin de caractériser les propriétés du volume d'échantillonnage (6) dans un secteur angulaire proche avant ;
      -- un ensemble de guidage (9, 10, 11), configuré pour guider le faisceau optique collimaté (34) de la source d'émission (3) vers les voies de mesure (4, 5, 7) au travers du volume d'échantillonnage ;

   - une partie pilotage (2), configurée pour contrôler les voies de mesure (4, 5, 7) pour effectuer les mesures de transmission et de diffusion de façon concomitante, et pour déterminer l'atténuation du faisceau optique collimaté (34) dans le volume d'échantillonnage (6) en fonction de l'intensité optique de référence ($I_0$), de l'intensité optique transmise et diffusée ($I_T$), et des intensités optiques diffusées ($I_D$).

**2.** Système de mesure selon la revendication 1, dans lequel l'ensemble de guidage (9, 10, 11) comprend une pluralité de séparateurs de faisceaux (9, 10) pour transmettre le faisceau optique collimaté (34) vers la voie de mesure de référence (4), vers la voie de mesure de transmission (5) et vers la voie de mesure de diffusion (7), et au moins un rétroréflecteur optique (11), disposé le long de l'axe optique de la source d'émission (3), et configuré pour replier sur lui-même le faisceau optique (34) ayant traversé le volume d'échantillonnage (6) en direction de la source d'émission (3).

**3.** Système de mesure selon l'une des revendications précédentes, dans lequel la voie de mesure de diffusion (7) comprend une lentille objectif de diffusion (12) et un capteur optoélectronique multiélément (13) configuré pour imager le plan de Fourier de la lentille objectif de diffusion (12).

**4.** Système de mesure selon la revendication 3, dans lequel un élément absorbant (15), configuré pour absorber le faisceau optique transmis et diffusé entre 0° et $\theta_{coll}$, recouvre les pixels de la partie centrale du capteur optoélectronique multiélément (13).

**5.** Système de mesure selon la revendication 4, dans lequel l'élément absorbant (15) a un taux d'absorption qui décroit depuis la partie centrale (16) du capteur optoélectronique multiélément (13) vers les extrémités du capteur optoélectronique multiélément (13).

**6.** Système de mesure selon la revendication 3, dans lequel le capteur optoélectronique multiélément (13) est dépourvu d'éléments d'acquisition optique dans sa partie centrale (16).

**7.** Système de mesure selon la revendication 3, dans lequel le capteur optoélectronique multiélément (13) a une dynamique telle qu'il puisse mesurer l'intensité transmise et l'intensité diffusée avec un rapport de puissance entre le signal de diffusion et le signal de transmission supérieur à $10^{-6}$.

**8.** Système de mesure selon l'une des revendications 3 à 7, dans lequel le capteur optoélectronique multiélément (13) est un capteur CMOS.

**9.** Système de mesure selon l'une des revendications 3 à 8, dans lequel la voie de mesure de diffusion (7) comprend un occulteur (17) disposé dans le plan focal de la lentille objectif de diffusion (12) et un montage composé d'au moins deux lentilles (18, 19), configuré pour imager le plan de Fourier de la lentille objectif de diffusion (12) sur le capteur optoélectronique multiélément (13) au travers d'un diaphragme de Lyot (20).

**10.** Système de mesure selon l'une des revendications précédentes, dans lequel la voie de mesure de transmission (5) comprend une lentille objectif de transmission (21), et un sténopé (22) de rayon $r_t$ placé à la distance focale $f_t$ de la lentille objectif de transmission (21), où $\tan(\theta_{coll}) = r_t / f_t$.

**11.** Système de mesure selon l'une des revendications précédentes, dans lequel la partie pilotage (2) est configurée pour :

- déterminer les valeurs de sections efficaces volumiques de diffusion du volume d'échantillonnage (6) à partir de l'intensité optique diffusée dans le volume d'échantillonnage ($I_D$) pour différents angles par rapport à l'axe optique du faisceau optique collimaté (34) ;
- appliquer un ajustement de courbe aux valeurs de sections efficaces volumiques de diffusion, et extrapoler la courbe obtenue aux valeurs de sections efficaces volumiques de diffusion entre 0° et $\theta_{coll}$ ;
- déterminer le coefficient d'extinction déduit de la mesure de transmission contaminée par la diffusion avant $\beta^*$ avec la relation suivante :

$$\beta^* = \beta - \int_0^{2\pi} \int_0^{\theta_{coll}} \mu(\theta, \varphi) \sin(\theta) d\theta \, d\varphi$$

Où $\beta$ correspond au coefficient d'extinction réelle calculée par la loi de Beer-Bouguer-Lambert, $\mu(\theta, \varphi)$ correspond à la valeur de sections efficaces volumiques qui varie en fonction de l'angle polaire $\theta$ et de l'angle azimutal de rotation autour de l'axe l'optique $\varphi$.

**12.** Système de mesure selon l'une des revendications précédentes, dans lequel la source d'émission (3), la voie de mesure de référence (4), la voie de mesure de transmission (5) et la voie de mesure de diffusion (7) sont intégrées dans un même boitier.

**13.** Procédé de détermination de l'atténuation d'une onde lumineuse traversant un volume d'échantillonnage, comprenant au moins une itération consistant à effectuer :

- une émission d'un faisceau optique collimaté (34) par une source d'émission (3) ($I_0$) ;
- une mesure d'une intensité optique de référence émise par la source d'émission (3) ($I_0$) ;
- une mesure d'une intensité optique transmise dans le volume d'échantillonnage ($I_T$) et diffusée dans le volume d'échantillonnage dans un intervalle d'angles polaires compris entre 0° et un angle de collection $\theta_{coll} > 0$, l'angle polaire étant défini rapport à l'axe optique du faisceau optique collimaté (34), où 0° indique la direction de propagation ;
- une mesure d'une intensité optique diffusée dans le volume d'échantillonnage ($I_D$), à une pluralité d'angles polaires par rapport à l'axe optique du faisceau optique collimaté (34) compris entre $\theta_{coll}$ et un angle de détection maximal $\theta_{max} > \theta_{coll}$, afin de caractériser les propriétés du volume d'échantillonnage (6) dans un secteur angulaire proche avant ;
- un contrôle des mesures réalisées de façon concomitante, et une détermination de l'atténuation en fonction de l'intensité optique de référence ($I_0$), de l'intensité optique transmise ($I_T$), et des intensités optiques diffusées ($I_D$).

**14.** Procédé selon la revendication 13, comprenant une pluralité d'itérations.

**15.** Procédé selon l'une des revendications 13 ou 14, dans lequel le volume d'échantillonnage (6) est un environnement nuageux, en particulier un nuage en phase de glace.

**Patentansprüche**

**1.** System zur Bestimmung der Dämpfung einer Lichtwelle, die ein Probenahmevolumen durchläuft, umfassend:

- einen Messabschnitt (1), umfassend:

-- eine Emissionsquelle (3) eines kollimierten optischen Strahls (34);
-- einen Referenzmesskanal (4), der konfiguriert ist, um eine von der Emissionsquelle (3) emittierte optische Referenzintensität ($I_0$) zu messen;
-- einen Transmissionsmesskanal (5), der konfiguriert ist, um eine transmittierte optische Intensität ($I_T$) im Probenahmevolumen (6) zu messen und im Probenahmevolumen (6) innerhalb eines Bereichs von Polarwinkeln zwischen 0° und einem Sammelwinkel $\theta_{coll} > 0$ diffundiert, wobei der Polarwinkel in Bezug auf die optische Achse des kollimierten optischen Strahls (34) definiert ist, wobei 0° die Ausbreitungsrichtung angibt;
-- einen Diffusionsmesskanal (7), der konfiguriert ist, um eine im Probenahmevolumen diffundierte optische Intensität ($I_D$) unter einer Vielzahl von Polarwinkeln in Bezug auf die optische Achse des kollimierten optischen Strahls (34) zwischen $\theta_{coll}$ und einem maximalen Detektionswinkel $\theta_{max} > \theta_{coll}$ zu messen, um die Eigenschaften des Probenahmevolumens (6) in einem winkelnahen Bereich zu charakterisieren;
-- eine Führungsanordnung (9, 10, 11), die konfiguriert ist, um den kollimierten optischen Strahl (34) von der Emissionsquelle (3) durch das Probenahmevolumen zu den Messkanälen (4, 5, 7) zu führen;

- einen Steuerungsabschnitt (2), der konfiguriert ist, um die Messkanäle (4, 5, 7) zu überprüfen, um gleichzeitig Transmissions- und Diffusionsmessungen durchzuführen und die Dämpfung des kollimierten optischen Strahls (34) im Probenahmevolumen (6) in Abhängigkeit von der optischen Referenzintensität ($I_0$), der transmittierten und diffundierten optischen Intensität ($I_T$) und den diffundierten optischen Intensitäten ($I_D$) zu bestimmen.

**2.** Messsystem nach Anspruch 1, wobei die Führungsanordnung (9, 10, 11) eine Vielzahl von Strahlteilern (9, 10) zur Transmission des kollimierten optischen Strahls (34) zum Referenzmesskanal (4), zum Transmissionsmesskanal (5) und zum Diffusionsmesskanal (7) umfasst, und mindestens einen optischen Retroreflektor (11), der entlang der optischen Achse der Emissionsquelle (3) angeordnet und konfiguriert ist, um den durch das Probenahmevolumen (6) hindurchgetretenen optischen Strahl (34) in Richtung der Emissionsquelle (3) auf sich selbst zurückzufalten.

3. Messsystem nach einem der vorstehenden Ansprüche, wobei der Diffusionsmesskanal (7) eine Diffusionsobjektivlinse (12) und einen optoelektronischen Mehrelementsensor (13) umfasst, der konfiguriert ist, um die Fourier-Ebene der Diffusionsobjektivlinse (12) abzubilden.

4. Messsystem nach Anspruch 3, wobei ein Absorptionselement (15), das konfiguriert ist, um den transmittierten und diffundierten optischen Strahl zwischen 0° und $\theta_{coll}$ zu absorbieren, die Pixel des mittleren Abschnitts des optoelektronischen Mehrelementsensors (13) abdeckt.

5. Messsystem nach Anspruch 4, wobei das Absorptionselement (15) eine Absorptionsrate aufweist, die vom mittleren Abschnitt (16) des optoelektronischen Mehrelementsensors (13) in Richtung der Enden des optoelektronischen Mehrelementsensors (13) hin abnimmt.

6. Messsystem nach Anspruch 3, wobei der optoelektronische Mehrelementsensor (13) in seinem mittleren Abschnitt (16) keine optischen Erfassungselemente aufweist.

7. Messsystem nach Anspruch 3, wobei der optoelektronische Mehrelementsensor (13) eine Dynamik aufweist, mit der er die transmittierte Intensität und die diffundierte Intensität mit einem Leistungsverhältnis zwischen dem diffundiertem Signal und dem transmittierten Signal von mehr als $10^{-6}$ messen kann.

8. Messsystem nach einem der Ansprüche 3 bis 7, wobei es sich bei dem optoelektronischen Mehrelementsensor (13) um einen CMOS-Sensor handelt.

9. Messsystem nach einem der Ansprüche 3 bis 8, wobei der Diffusionsmesskanal (7) einen in der Brennebene der Diffusionsobjektivlinse (12) angeordneten Verschluss (17) und eine Anordnung aus mindestens zwei Linsen (18, 19) umfasst, die konfiguriert ist, um die Fourier-Ebene der Diffusionsobjektivlinse (12) durch eine Lyot-Blende (20) auf den optoelektronischen Mehrelementsensor (13) abzubilden.

10. Messsystem nach einem der vorstehenden Ansprüche, wobei der Transmissionsmesskanal (5) eine Transmissionsobjektivlinse (21) und eine Lochblende (22) mit Radius $r_t$ umfasst, die im Brennpunktabstand $f_t$ der Transmissions-objektivlinse (21) platziert ist, wobei $\tan(\theta_{coll}) = r_t / f_t$.

11. Messsystem nach einem der vorstehenden Ansprüche, wobei der Steuerungsabschnitt (2) konfiguriert ist zum:

- Bestimmen der Werte der volumetrischen Wirkungsquerschnitte der Diffusion des Probenahmevolumens (6) anhand der im Probenahmevolumen diffundierten optischen Intensität ($I_D$) für unterschiedliche Winkel relativ zur optischen Achse des kollimierten optischen Strahls (34);
- Anwenden einer Kurvenanpassung auf die Werte der volumetrischen Wirkungsquerschnitte der Diffusion, und Extrapolieren der resultierenden Kurve auf die Werte der volumetrischen Wirkungsquerschnitte der Diffusion zwischen 0° und $\theta_{coll}$;
- Bestimmen des Extinktionskoeffizienten, der aus der durch Diffusion kontaminierten Transmissionmessung abgeleitet ist, vor $\beta^*$ mit folgender Beziehung:

$$\beta^* = \beta - \int_0^{2\pi} \int_0^{\theta_{coll}} \mu(\theta, \varphi) \sin(\theta) d\theta \, d\varphi$$

wobei $\beta$ dem tatsächlichen Extinktionskoeffizienten entspricht, der nach dem Beer-Bouguer-Lambert-Gesetz be-rechnet wird, $\mu(\theta, \varphi)$ dem Wert der volumetrischen Wirkungsquerschnitte entspricht, der sich je nach dem Polarwinkel $\theta$ und dem Azimutwinkel der Drehung um die optische Achse $\varphi$ ändert.

12. Messsystem nach einem der vorstehenden Ansprüche, wobei die Emissionsquelle (3), der Referenzmesskanal (4), der Transmissionsmesskanal (5) und der Diffusionsmesskanal (7) in demselben Gehäuse integriert sind.

13. Verfahren zur Bestimmung der Dämpfung einer Lichtwelle, die ein Probenahmevolumen durchläuft, umfassend mindestens eine Iteration, bestehend aus der Durchführung von:

- einer Emission eines kollimierten optischen Strahls (34) durch eine Emissionsquelle (3) ($I_0$);

- einer Messung einer optischen Referenzintensität, die von der Emissionsquelle (3) ($I_\theta$) emittiert wird;
- einer Messung einer im Probenahmevolumen ($I_T$) transmittierten und im Probenahmevolumen in einem Intervall von Polarwinkeln zwischen 0° und einem Sammelwinkel $\theta_{coll} > 0$ diffundierten optischen Intensität, wobei der Polarwinkel in Bezug auf die optische Achse des kollimierten optischen Strahls (34) definiert ist, wobei 0° die Ausbreitungsrichtung angibt;
- einer Messung einer im Probenahmevolumen diffundierten optischen Intensität ($I_D$) bei einer Vielzahl von Polarwinkeln in Bezug auf die optische Achse des kollimierten optischen Strahls (34) zwischen $\theta_{coll}$ und einem maximalen Detektionswinkel $\theta_{max} > \theta_{coll}$, um die Eigenschaften des Probenahmevolumens (6) in einem nahen vorderen Winkelbereich zu charakterisieren;
- einer Überprüfung der gleichzeitig durchgeführten Messungen und einer Bestimmung der Dämpfung in Abhängigkeit von der optischen Referenzintensität ($I_0$),der transmittierten optischen Intensität ($I_T$) und der diffundierten optischen Intensitäten ($I_D$).

14. Verfahren nach Anspruch 13, umfassend eine Vielzahl von Iterationen.

15. Verfahren nach einem der Ansprüche 13 oder 14, wobei es sich bei dem Probenahmevolumen (6) um eine trübe Umgebung, insbesondere um eine Wolke in der Eisphase, handelt.

**Claims**

1. A system for determining the attenuation of a light wave passing through a sampling volume, comprising:

   - a measurement part (1) comprising:

     -- an emission source (3) emitting a collimated optical beam (34);
     -- a reference measurement channel (4), configured to measure a reference optical intensity emitted by the emission source (3) ($I_0$);
     -- a transmission measurement channel (5), configured to measure a transmitted optical intensity ($I_T$) in the sampling volume (6) and scattered in the sampling volume (6) in a polar angle range between 0° and a collection angle $\theta_{coll} > 0$, the polar angle being defined with respect to the optical axis of the collimated optical beam (34), where 0° indicates the direction of propagation;
     -- a scattering measurement channel (7), configured to measure a scattered optical intensity in the sampling volume ($I_D$), at a plurality of polar angles relative to the optical axis of the collimated optical beam (34) between $\theta_{coll}$ and a maximum detection angle $\theta_{max} > \theta_{coll}$, in order to characterize the properties of the sampling volume (6) in a near forward angular sector;
     -- a guide assembly (9, 10, 11), configured to guide the collimated optical beam (34) from the emission source (3) to the measurement channels (4, 5, 7) through the sampling volume;

   - a control part (2), configured to control the measurement channels (4, 5, 7) in order to carry out transmission and scattering measurements concomitantly, and to determine the attenuation of the collimated optical beam (34) in the sampling volume (6) according to the reference optical intensity ($I_0$), the transmitted and scattered optical intensity ($I_T$), and the scattered optical intensities ($I_D$).

2. The measurement system according to claim 1, wherein the guide assembly (9, 10, 11) comprises a plurality of beam splitters (9, 10) for transmitting the collimated optical beam (34) to the reference measurement channel (4), to the transmission measurement channel (5) and to the scattering measurement channel (7), and at least one optical retroreflector (11), arranged along the optical axis of the emission source (3), and configured to fold the optical beam (34) back on itself that has passed through the sampling volume (6) in the direction of the emission source (3).

3. The measurement system according to either of the preceding claims, wherein the scattering measurement channel (7) comprises a scattering objective lens (12) and a multi-element optoelectronic sensor (13) configured to image the Fourier plane of the scattering objective lens (12).

4. The measurement system according to claim 3, wherein an absorbing element (15), configured to absorb the optical beam transmitted and scattered between 0° and $\theta_{coll}$, covers the pixels of the central part of the multi-element optoelectronic sensor (13).

5. The measurement system according to claim 4, wherein the absorbing element (15) has an absorption rate that decreases from the central part (16) of the multi-element optoelectronic sensor (13) towards the ends of the multi-element optoelectronic sensor (13).

6. The measurement system according to claim 3, wherein the multi-element optoelectronic sensor (13) is devoid of optical acquisition elements in its central part (16).

7. The measurement system according to claim 3, wherein the multi-element optoelectronic sensor (13) has a dynamic range such that it can measure the transmitted and scattered intensity with a power ratio between the scattering signal and the transmission signal greater than $10^{-6}$.

8. The measurement system according to one of claims 3 to 7, wherein the multi-element optoelectronic sensor (13) is a CMOS sensor.

9. The measuring system according to one of claims 3 to 8, wherein the scattering measurement channel (7) comprises a light-blocking device (17) arranged in the focal plane of the scattering objective lens (12) and an assembly of at least two lenses (18, 19), configured to image the Fourier plane of the scattering objective lens (12) onto the multi-element optoelectronic sensor (13) through a Lyot diaphragm (20).

10. The measuring system according to one of the preceding claims, wherein the transmission measurement channel (5) comprises a transmission objective lens (21), and a pinhole (22) of radius $r_t$ placed at focal distance $f_t$ of the transmission objective lens (21), where $\tan(\theta_{coll}) = r_t / f_t$.

11. The measurement system according to one of the preceding claims, wherein the control part (2) is configured to:

- determine the effective volume scattering cross-sections of the sampling volume (6) from the optical intensity scattered in the sampling volume ($I_D$) at different angles to the optical axis of the collimated optical beam (34);
- apply a curve adjustment to the scattering effective volume cross-section values, and extrapolate the curve obtained to the scattering effective volume cross-section values between 0° and $\theta_{coll}$;
- determine the extinction coefficient deduced from the transmission measurement

contaminated by scattering before β*with the following relationship:

$$\beta^{*} = \beta - \int_{0}^{2\pi} \int_{0}^{\theta_{coll}} \mu(\theta, \varphi) \sin(\theta) d\theta \, d\varphi$$

Where β corresponds to the real extinction coefficient calculated by the Beer-Bouguer-Lambert law, $\mu(\theta, \varphi)$ corresponds to the value of effective volume cross-sections that varies as a function of the polar angle θ and the azimuthal angle of rotation around the optical axis φ.

12. The measurement system according to one of the preceding claims, wherein the emission source (3), the reference measurement channel (4), the transmission measurement channel (5) and the scattering measurement channel (7) are integrated in a single housing.

13. A method of determining the attenuation of a light wave passing through a sampling volume, comprising at least one iteration consisting in carrying out:

- emission of a collimated optical beam (34) by an emission source (3) ($I_0$);
- a measurement of a reference optical intensity emitted by the emission source (3) ($I_0$);
- a measurement of an optical intensity transmitted into the sampling volume ($I_T$) and scattered in the sampling volume in a polar angle range between 0° and a collection angle $\theta_{coll} > 0$, the polar angle being defined relative to the optical axis of the collimated optical beam (34), where 0° indicates the direction of propagation;
- a measurement of an optical intensity scattered in the sampling volume ($I_D$), at a plurality of polar angles to the optical axis of the collimated optical beam (34) between $\theta_{coll}$ and a maximum detection angle $\theta_{max} > \theta_{coll}$, in order to characterize the properties of the sampling volume (6) in a near forward angular sector;
- a check of the measurements carried out concomitantly, and a determination of the attenuation as a function of the reference optical intensity ($I_0$), the transmitted optical intensity ($I_T$), and the scattered optical intensities ($I_D$).

**14.** The method according to claim 13, comprising a plurality of iterations.

**15.** The method according to one of claims 13 or 14, wherein the sampling volume (6) is a cloudy environment, in particular an ice-phase cloud.

# Fig. 1

# Fig. 2

EP 4 544 287 B1

# Fig. 3

# Fig. 4

18

EP 4 544 287 B1

# Fig. 5

# Fig. 6

# Fig. 7

19

## RÉFÉRENCES CITÉES DANS LA DESCRIPTION

### Documents brevets cités dans la description

- US 2009103085 A1

### Littérature non-brevet citée dans la description

- **AUSTIN, R. W.** ; **T. J. PETZOLD**. An Instrument For The Measurement Of Spectral Attenuation Coefficient And Narrow Angle Volume Scattering Function Of Ocean Waters. *Proc. SPIE 0064, Ocean Optics*, 10 November 1975, vol. IV